# FASCICULE DE BREVET EUROPEEN

(11) **EP 1 120 405 B1**
(45) Date de publication et mention de la délivrance du brevet: **27.10.2004**
(21) Numéro de dépôt: 01400112.7
(22) Date de dépôt: 16.01.2001
(51) Int. Cl.: C07C 281/16, C07C 281/18, A61K 7/13

(54) **Nouvelles bases d'oxydation à chaîne guanidine, leur procédé de préparation, leur utilisation pour la teinture d'oxydation des fibres kératiniques, compositions tinctoriales et procédés de teinture**
Oxidationsbasen mit Guanidin Kette, Verfahren zu deren Herstellung, deren Verwendung zur oxidativen Färbung von keratinischen Fasern, Farbmitteltzusammensetzung und Färbeverfahren
Oxidation bases with a guanidine chain, process for their preparation, their use for oxidation dyeing of keratinous fibers, dyeing compositions and dyeing processes

(30) Priorité: 27.01.2000 FR 0001055
(43) Date de publication de la demande: 01.08.2001
(73) Titulaire: L'OREAL, 75008 Paris (FR)
(72) Inventeur: Bordier, Thierry, 93290 Tremblay (FR); Philippe, Michel, 91320 Wissous (FR)
(74) Mandataire: Fevrier, Murielle

(56) Documents cités:
- EP-A- 0 918 053

## Description

L'invention a pour objet de nouvelles bases d'oxydation à noyau benzénique et comportant une chaîne guanidine, leur procédé de préparation, leur utilisation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, ou bien encore des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

De nombreux brevets décrivant de nouveaux colorants capillaires à noyau benzénique ont été publiés dans la littérature. Parmi les plus récents, on peut notamment citer les dérivés de pyrrolidine décrits dans le brevet US 5,851,237, les dérivés de polyol décrits dans la demande de brevet JP 11-158046 ou encore les dérivés de pipérazine tels que ceux décrits dans la demande de brevet DE 197 28 335.

Cependant, dans le domaine des colorants d'oxydation, les propriétés tinctoriales telles que la ténacité vis à vis des diverses agressions et des divers traitements que peuvent subir les fibres kératiniques, ainsi que la variété des nuances obtenues après réaction avec différents coupleurs restent toujours à améliorer.

Or, la demanderesse vient maintenant de découvrir, de façon totalement inattendue et surprenante, que de nouveaux composés à noyau benzénique comportant une chaîne guanidine, de formule (I) ci-après définie, non seulement conviennent pour une utilisation comme base d'oxydation, mais en outre qu'ils permettent d'obtenir des compositions tinctoriales conduisant à des colorations puissantes, dans une large palette de couleurs, et présentant d'excellentes propriétés de résistances, (ténacité), aux différents traitements que peuvent subir les fibres kératiniques. Cette ténacité est significativement supérieure à l'état de la technique.

Ces découvertes sont à la base de la présente invention.

L'invention a donc pour premier objet de nouveaux composés de formule (I) suivante, et leurs sels d'addition avec un acide : 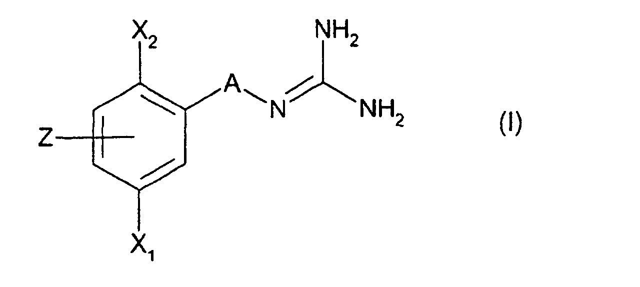 dans laquelle :
- X₁ et X₂ représentent un radical hydroxyle, un radical -NHR₁ ou -NR₁R₂, étant entendu que X₁ et X₂ ne peuvent pas représenter simultanément un radical hydroxyle ;
- R₁ et R₂, identiques ou différents, représentent un atome d'hydrogène, un radical alkyle en C₁-C₈, monohydroxyalkyle en C₁-C₈, polyhydroxyalkyle en C₂-C₈, aminoalkyle en C₂-C₈, monoalkyl(C₁-C₄)aminoalkyle en C₁-C₄, dialkyl(C₁-C₄)aminoalkyle en C₁-C₄, ou halogénoalkyle en C₁-C₈ ; lorsque X₁ et/ou X₂ représente(nt) un radical -NR₁R₂, alors R₁ et R₂ peuvent également former ensemble, conjointement avec l'atome d'azote auquel ils sont liés, un cycle à 4, 5 ou 6 chaînons, aromatique ou non ;
- R₁ et R₂, indépendamment l'un de l'autre, peuvent également représenter un cycle à 4, 5 ou 6 chaînons, aromatique ou non, ledit cycle pouvant renfermer un ou plusieurs hétéroatomes ;
- A représente un radical divalent choisi parmi -CH=N- et -CH₂-NH- ;
- Z représente un atome d'hydrogène, un atome d'halogène, un cycle aromatique ou non ayant 4, 5 ou 6 chaînons, un radical alkyle en C₁-C₈, monohydroxyalkyle en C₁-C₈, polyhydroxyalkyle en C₂-C₈, aminoalkyle en C₂-C₈, monoalkyl(C₁-C₄)aminoalkyle en C₁-C₄, dialkyl(C₁-C₄)aminoalkyle en C₁-C₄, ou halogénoalkyle en C₁-C₈, cyano, ou un groupement -BR₃ dans lequel B représente un radical divalent choisi parmi les radicaux suivants :

   ―CH=N― ;

   ―CH₂-NH- ;

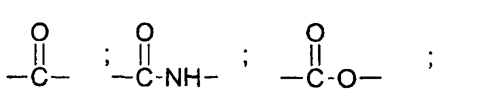

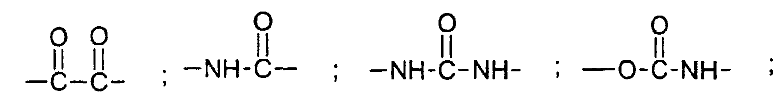

   ―S― ;

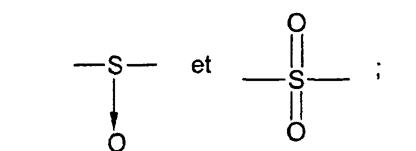

   et dans lequel R₃ représente un radical alkyle en C₁-C₈, monohydroxyalkyle en C₁-C₈, polyhydroxyalkyle en C₂-C₈, aminoalkyle en C₂-C₈, monoalkyl(C₁-C₄)aminoalkyle en C₁-C₄, dialkyl(C₁-C₄)aminoalkyle en C₁-C₄, ou halogénoalkyle en C₁-C₈.

Comme indiqué précédemment, les colorations obtenues avec le ou les composés de formule (I) conforme à l'invention sont puissantes et permettent d'atteindre une large palette de couleurs. Elles présentent de plus d'excellentes propriétés de résistance vis à vis de l'action des différents agents extérieurs (lumière, intempéries, lavage, ondulation permanente, transpiration, frottements).

Dans la formule (I) ci-dessus les radicaux alkyle peuvent être linéaires ou ramifiés.

Dans la formule (I) ci-dessus, lorsque R₁ et R₂ forment, conjointement avec l'atome d'azote auquel ils sont liés, un cycle à 4, 5 ou 6 chaînons, ou lorsque R₁ et/ou R₂ représentent un cycle à 4, 5 ou 6 chaînons, ou lorsque Z représente un cycle aromatique ou non à 4, 5 ou 6 chaînons, alors lesdits cycles peuvent porter un ou plusieurs substituants pouvant par exemple être choisis parmi les groupements suivants: un radical hydroxyle, un radical amido, un atome d'halogène, un radical cyano, un radical alkyle en C₁-C₈, un cycle aromatique ou non en C₄, C₅ ou C₆.

Parmi les composés de formule (I) ci-dessus, on peut notamment citer :
- la 5-amino-2-hydroxy-benzylidèneamino-guanidine,
- la 2-amino-5-hydroxy-benzylidèneamino-guanidine,
- la 2,5-diamino-benzylidèneamino-guanidine,
- la 5-dihydroxyéthylamino-2-hydroxy-benzylidèneamino-guanidine,
- la 5-diméthylamino-2-hydroxy-benzylidèneamino-guanidine,
- la 2-dihydroxyéthylamino-5-hydroxy-benzylidèneamino-guanidine,
- la 2-diméthylamino-5-hydroxy-benzylidèneamino-guanidine,
- la 2-dihydroxyéthylamino-5-amino-benzylidèneamino-guanidine,
- la 5-dihydroxyéthylamino-2-amino-benzylidèneamino-guanidine,
- la 5-diméthylamino-2-amino-benzylidèneamino-guanidine,
- la 2-diméthylamino-5-amino-benzylidèneamino-guanidine,
- la 2-N-pyrrolidinyl-5-amino-benzylidèneamino-guanidine,
- la 5-N-pyrrolidinyl-2-amino-benzylidèneamino-guanidine,
et leurs sels d'addition avec un acide.

Les sels d'addition avec un acide des composés de formule (I) conforme à l'invention, (mono- ou di salification), sont de préférence choisis parmi les sels inorganiques ou organiques tels que les chlorhydrates, les bromhydrates, les sulfates, les citrates, les succinates, les tartrates, les lactates et les acétates. Les chlorhydrates sont particulièrement préférés.

L'invention a également pour objet le procédé de préparation des composés de formule (I) conformes à l'invention consistant à réaliser un couplage direct de l'amino guanidine sur un dérivé benzaldéhyde dont les substituants correspondent au composé de formule (I) que l'on souhaite obtenir, dans un solvant organique tel qu'un alcool comme l'éthanol à une température comprise entre 10°C et la température de reflux dudit solvant. Ledit couplage peut être suivi d'autres réactions, notamment d'une réduction classique d'un groupement nitro ou d'une réduction d'une fonction imine, conduisant aux composés de formule (I) attendus. Les détails seront donnés dans la partie expérimentale ci-dessous.

Lorsque la synthèse est terminée, les composés de formule (I) conformes à l'invention peuvent, le cas échéant, être récupérés par des méthodes bien connues de l'état de la technique telles que la cristallisation, la distillation.

Un autre objet de l'invention est l'utilisation des composés de formules (I) conformes à l'invention à titre de base d'oxydation pour la teinture d'oxydation des fibres kératiniques, et en particulier des fibres humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de base d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 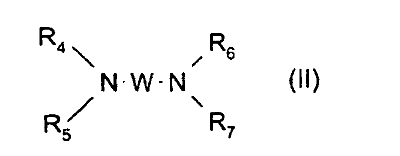 dans laquelle W est un reste propylène éventuellement substitué par un groupement hydroxyle ou un radical alkyle en C₁-C₆ ; R₄, R₅, R₆ et R₇, identiques ou différents, représentent un atome d'hydrogène, un radical alkyle en C₁-C₆ ou hydroxyalkyle en C₁-C₆.

La composition tinctoriale conforme à l'invention peut encore contenir, en plus du ou des composés de formule (I) définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines différentes des composés de formule (I), les bis-phénylalkylènediamines, les para-aminophénols différents des composés de formule (I), les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]-pyrimidin-7-yl)-(2-hydroxy-éthyl)-amino]-éthanol, le 2-[(7-amino-pyrazolo[1,5-a]-pyrimidin-3-yl)-(2-hydroxy-éthyl)-amino]-éthanol, la 5,6-diméthyl pyrazolo[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, 6-hydroxybenzomorpholine, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation additionnelles et coupleurs) sont notamment choisis parmi les chlorhydrates, les bromhydrates, les sulfates, les citrates, les succinates, les tartrates, les lactates et les acétates.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée séparément, simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, et les enzymes parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES DE PREPARATION

### EXEMPLE DE PREPARATION 1 : Synthèse du dichlorhydrate de 5-amino-2-hydroxy-benzylidèneamino-guanidine

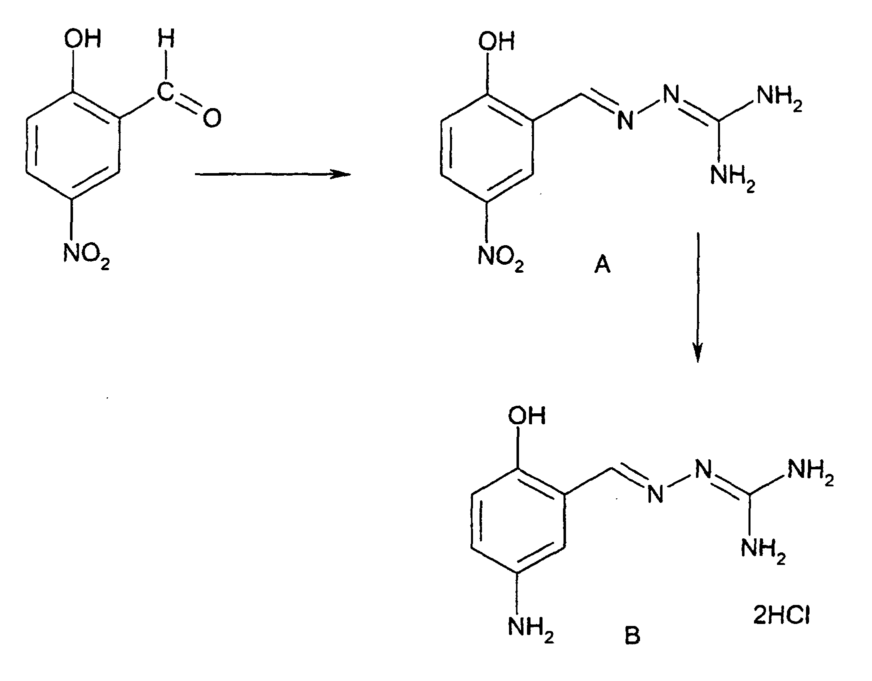

### a) Préparation du 2-hydroxy-5-nitro-benzylidèneamino-guanidine monohydrate (A)

Dans un réacteur de 500 ml, on a dissous 9 g (53,85 mmole) de 2-hydroxy-5-nitro-benzaldéhyde dans 200 ml d'éthanol. Puis, on a introduit 5,95 g (53,85 mmole) d'amino guanidine hydrochlorure et 7,6 ml de triéthylamine. Le mélange homogène a été chauffé à 45°C pendant 3 heures. Un précipité jaune s'est formé. Après filtration, lavage à l'eau et à l'acétone, puis séchage sous vide sur pentaoxyde de phosphore, 12 g d'un solide jaune clair ont été obtenus avec un rendement final en monohydrate de 92%.
Point de fusion : supérieur à 140°C
Analyse élémentaire calculée pour C₈H₉N₅O₃, H₂O :

| % | C | H | N | O |
|---|---|---|---|---|
| Calculé | 39,80 | 4,56 | 29,02 | 26,53 |
| Trouvé | 39,51 | 4,82 | 28,38 | 25,15 |

### b) Préparation du dichlorhydrate de 5-amino-2-hydroxy-benzylidèneaminoguanidine (B)

Le composé A obtenu ci-dessus à l'étape précédente (30 g, 134,4 mmole) a été mis en suspension dans 600 ml d'éthanol en présence de palladium sur charbon sec, puis hydrogéné sous 15 bar à une température de 40°-60°C. Le mélange a été filtré sous azote, puis récupéré dans une solution d'éthanol chlorhydrique glacée. Après cristallisation, filtration, lavage à l'éther éthylique puis séchage sous vide, on a obtenu 9,9 g d'un solide blanc avec un rendement final de 28% qui ont fondu à 215°C et dont l'analyse élémentaire calculée pour C₈H₁₁N₅O, 2HCl, 1H₂O était :

| % | C | H | N | O | Cl |
|---|---|---|---|---|---|
| Calculé | 33,79 | 5,27 | 24,64 | 11,26 | 24,99 |
| Trouvé | 34,35 | 5,24 | 24,88 | 10,06 | 25,38 |

### EXEMPLE DE PREPARATION 2 : Synthèse du dichlorhydrate de 2-amino-5-hydroxy-benzylidèneamino-guanidine

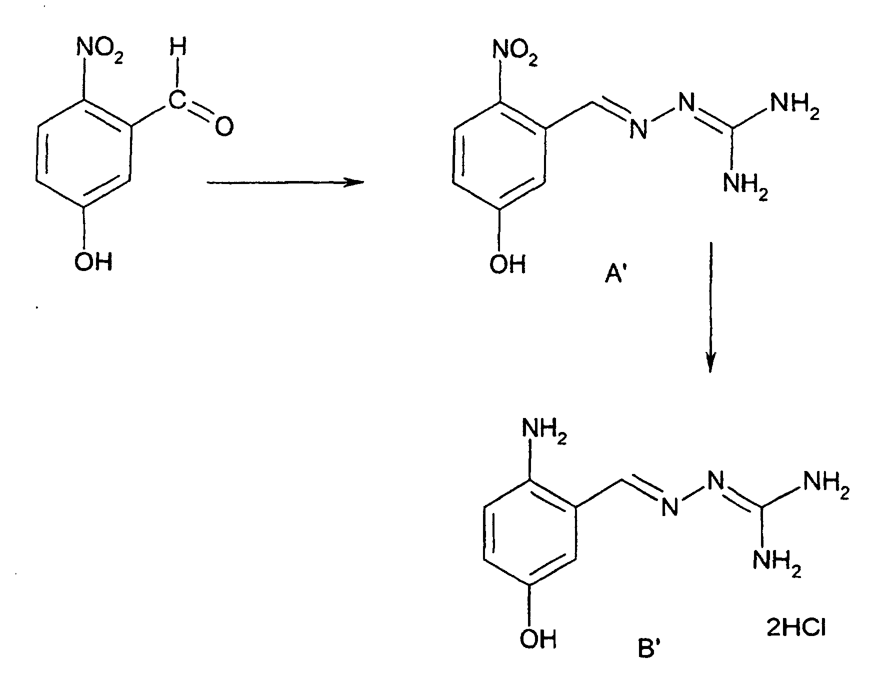

### a) Préparation du 5-hydroxy-2-nitro-benzylidèneamino-guanidine monohydrate (A')

Dans un réacteur de 100 ml, on a dissout 4,8 g (28,7 mmole) de 5-hydroxy-2-nitro-benzaldéhyde dans 40 ml de méthanol. On a ensuite introduit 3,2 g (28,7 mmole) d'amino guanidine hydrochlorure et 4 ml de triéthylamine. Le mélange homogène a été chauffé à une température de 45-50°C pendant environ 24 heures. Un précipité jaune s'est formé. Après filtration, lavage à l'eau et à l'acétone puis séchage sous vide sur pentaoxyde de phosphore, 5,5 g d'un solide jaune clair ont été obtenus avec un rendement final de 85%.

L'analyse élémentaire calculée pour C₈H₉N₅O₃ était :

| % | C | H | N | O |
|---|---|---|---|---|
| Calculé | 43,05 | 4,06 | 31,38 | 21,51 |
| Trouvé | 42,64 | 3,91 | 30,67 | 21,82 |

### b) Préparation du dichlorhydrate de 2-amino-5-hydroxy-benzylidèneaminoguanidine, (B')

Le composé nitré A' obtenu ci-dessus à l'étape précédente (3 g, 13,4 mmole) a été réduit selon la méthode décrite ci-dessus à l'exemple 1, étape b), par hydrogénation catalytique sous une pression de 15 bars à la température de 40°C, pour donner 2 g (56%) de composé B'.

L'analyse élémentaire calculée pour C₈H₁₁N₅0, 2HCI était :

| % | C | H | N | O | Cl |
|---|---|---|---|---|---|
| Calculé | 36,11 | 4,92 | 26,32 | 6,01 | 26,64 |
| Trouvé | 35,86 | 4,94 | 26,05 | 7,62 | 26,37 |

### EXEMPLES D'APPLICATION

### EXEMPLES 1 à 6 DE TEINTURE EN MILIEU BASIQUE

On a préparé les compositions tinctoriales suivantes (teneurs en grammes) :

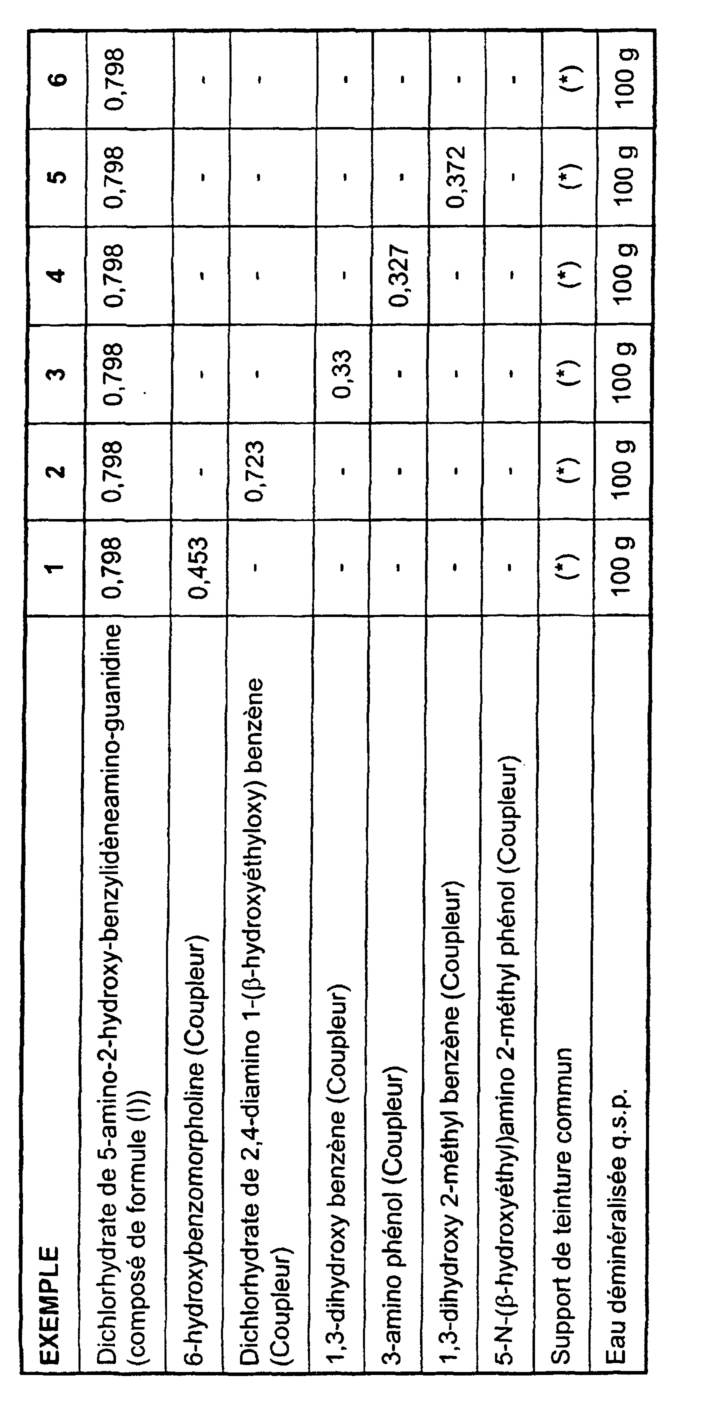

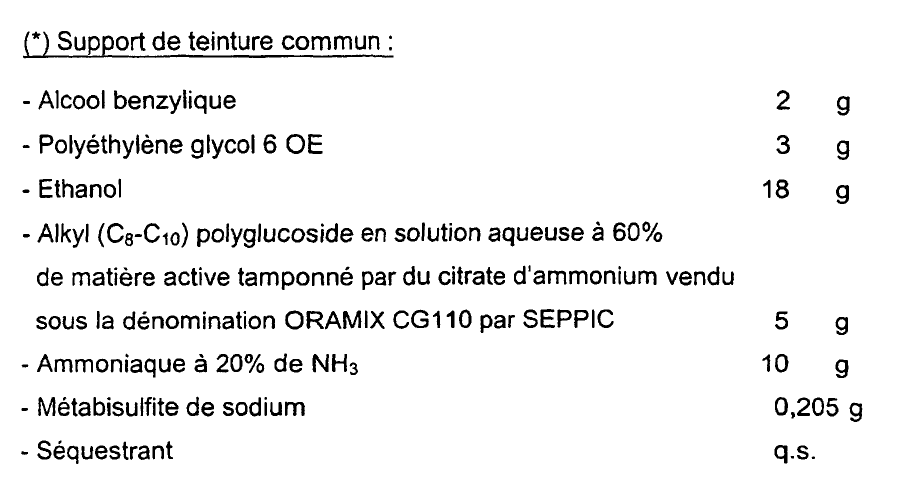

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de TEINTURE** | **Nuance sur cheveux naturels** |
|---|---|---|
| **1** | 10 ± 0,2 | Blond foncé doré acajou |
| **2** | 10 ± 0,2 | Châtain cendré irisé |
| **3** | 10 ± 0,2 | Blond foncé doré mat |
| **4** | 10 ± 0,2 | Blond acajou cendré |
| **5** | 10 ± 0,2 | Acajou |
| **6** | 10 ± 0,2 | Blond foncé cuivré |

## Revendications

1. Composés de formule (I) suivante, et leurs sels d'addition avec un acide : 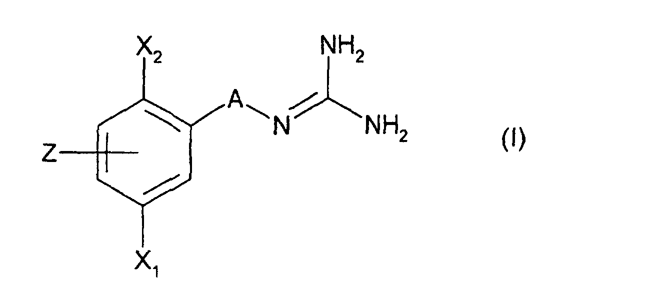 dans laquelle :
- X₁ et X₂ représentent un radical hydroxyle, un radical -NHR₁ ou -NR₁R₂, étant entendu que X₁ et X₂ ne peuvent pas représenter simultanément un radical hydroxyle ;
- R₁ et R₂, identiques ou différents, représentent un atome d'hydrogène, un radical alkyle en C₁-C₈, monohydroxyalkyle en C₁-C₈, polyhydroxyalkyle en C₂-C₈, aminoalkyle en C₂-C₈, monoalkyl(C₁-C₄)aminoalkyle en C₁-C₄, dialkyl(C₁-C₄)aminoalkyle en C₁-C₄, ou halogénoalkyle en C₁-C₈ ; lorsque X₁ et/ou X₂ représente(nt) un radical -NR₁R₂, alors R₁ et R₂ peuvent également former ensemble, conjointement avec l'atome d'azote auquel ils sont liés, un cycle à 4, 5 ou 6 chaînons, aromatique ou non ;
- R₁ et R₂, indépendamment l'un de l'autre, peuvent également représenter un cycle à 4, 5 ou 6 chaînons, aromatique ou non, ledit cycle pouvant renfermer un ou plusieurs hétéroatomes ;
- A représente un radical divalent choisi parmi -CH=N- et -CH₂-NH- ;
- Z représente un atome d'hydrogène, un atome d'halogène, un cycle aromatique ou non ayant 4, 5 ou 6 chaînons, un radical alkyle en C₁-C₈, monohydroxyalkyle en C₁-C₈, polyhydroxyalkyle en C₂-C₈, aminoalkyle en C₂-C₈, monoalkyl(C₁-C₄)aminoalkyle en C₁-C₄, dialkyl(C₁-C₄)aminoalkyle en C₁-C₄, ou halogénoalkyle en C₁-C₈, cyano, ou un groupement -BR₃ dans lequel B représente un radical divalent choisi parmi les radicaux suivants :
―CH=N― ;
―CH₂-NH- ; 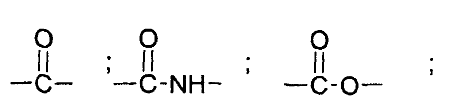 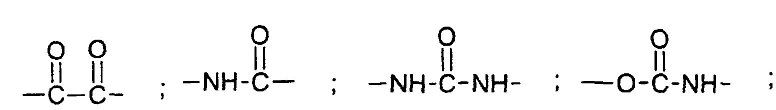
―S― ; 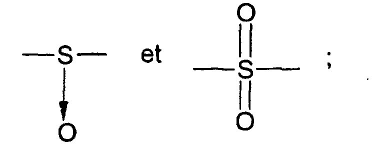
et dans lequel R₃ représente un radical alkyle en C₁-C₈, monohydroxyalkyle en C₁-C₈, polyhydroxyalkyle en C₂-C₈, aminoalkyle en C₂-C₈, monoalkyl(C₁-C₄)aminoalkyle en C₁-C₄, dialkyl(C₁-C₄)aminoalkyle en C₁-C₄, ou halogénoalkyle en C₁-C₈.

2. Composés selon la revendication 1, **caractérisés par le fait que** lorsque R₁ et R₂ forment, conjointement avec l'atome d'azote auquel ils sont liés, un cycle à 4, 5 ou 6 chaînons, ou lorsque R₁ et/ou R₂ représentent un cycle à 4, 5 ou 6 chaînons, ou lorsque Z représente un cycle aromatique ou non à 4, 5 ou 6 chaînons, alors lesdits cycles peuvent porter un ou plusieurs substituants choisis parmi les groupements suivants : un radical hydroxyle, un radical amido, un atome d'halogène, un radical cyano, un radical alkyle en C₁-C₈, un cycle aromatique ou non en C₄, C₅ ou C₆.

3. Composés selon la revendication 1 ou 2, **caractérisés par le fait qu'**ils sont choisis parmi :
- la 5-amino-2-hydroxy-benzylidèneamino-guanidine,
- la 2-amino-5-hydroxy-benzylidèneamino-guanidine,
- la 2,5-diamino-benzylidèneamino-guanidine,
- la 5-dihydroxyéthylamino-2-hydroxy-benzylidèneamino-guanidine,
- la 5-diméthylamino-2-hydroxy-benzylidèneamino-guanidine,
- la 2-dihydroxyéthylamino-5-hydroxy-benzylidèneamino-guanidine,
- la 2-diméthylamino-5-hydroxy-benzylidèneamino-guanidine,
- la 2-dihydroxyéthylamino-5-amino-benzylidèneamino-guanidine,
- la 5-dihydroxyéthylamino-2-amino-benzylidèneamino-guanidine,
- la 5-diméthylamino-2-amino-benzylidèneamino-guanidine,
- la 2-diméthylamino-5-amino-benzylidèneamino-guanidine,
- la 2-N-pyrrolidinyl-5-amino-benzylidèneamino-guanidine,
- la 5-N-pyrrolidinyl-2-amino-benzylidèneamino-guanidine,
et leurs sels d'addition avec un acide.

4. Composés selon l'une quelconque des revendications précédentes, **caractérisés par le fait que** les sels d'addition avec un acide des composés de formule (I) sont choisis parmi les chlorhydrates, les bromhydrates, les sulfates, les citrates, les succinates, les tartrates, les lactates et les acétates.

5. Procédé de préparation des composés de formule (I) tels que définis à l'une quelconque des revendications 1 à 4, consistant à réaliser un couplage direct de l'amino guanidine sur un dérivé benzaldéhyde dont les substituants correspondent au composé de formule (I) que l'on souhaite obtenir, dans un solvant organique, à une température comprise entre 10°C et la température de reflux dudit solvant.

6. Utilisation des composés de formules (I) tels que définis à l'une quelconque des revendications 1 à 4, à titre de base d'oxydation pour la teinture d'oxydation des fibres kératiniques et en particulier des fibres humaines telles que les cheveux.

7. Composition pour la teinture d'oxydation des fibres kératiniques et en particulier des fibres kératiniques humaines telles que les cheveux, **caractérisée par le fait qu'**elle comprend, à titre de base d'oxydation, dans un milieu approprié pour la teinture, au moins un composé de formule (I) tel que défini à l'une quelconque des revendications 1 à 4.

8. Composition selon la revendication 7, **caractérisée par le fait que** le ou les composés de formule (I) représentent de 0,0005 à 12 % en poids du poids total de la composition tinctoriale.

9. Composition selon la revendication 8, **caractérisée par le fait que** le ou les composés de formule (I) représentent de 0,005 à 6 % en poids du poids total de la composition tinctoriale.

10. Composition selon l'une quelconque des revendications 7 à 9, **caractérisée par le fait qu'**elle contient, en plus du ou des composés de formule (I), au moins une base d'oxydation additionnelle choisie parmi les paraphénylènediamines différentes des composés de formule (I), les bis-phénylalkylènediamines, les para-aminophénols différents des composés de formule (I), les ortho-aminophénols et les bases hétérocycliques.

11. Composition selon la revendication 10, **caractérisée par le fait que** les paraphénylènediamines sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl) amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

12. Composition selon la revendication 10, **caractérisée par le fait que** les bis-phénylalkylènediamines sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,5-dioxaoctane, et leurs sels d'addition avec un acide.

13. Composition selon la revendication 10, **caractérisée par le fait que** les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

14. Composition selon la revendication 10, **caractérisée par le fait que** les bases hétérocycliques sont choisies parmi les dérivés pyridiniques, les dérivés pyrimidiniques et les dérivés pyrazoliques.

15. Composition selon l'une quelconque des revendications 7 à 14, **caractérisée par le fait que** la ou les bases d'oxydation additionnelles représentent de 0,0005 à 12 % en poids du poids total de la composition tinctoriale.

16. Composition selon la revendication 15, **caractérisée par le fait que** la ou les bases d'oxydation additionnelles représentent de 0,005 à 6 % en poids du poids total de la composition tinctoriale.

17. Composition selon l'une quelconque des revendications 7 à 16, **caractérisée par le fait qu'**elle renferme au moins un coupleur et/ou au moins un colorant direct.

18. Composition selon la revendication 17, **caractérisée par le fait que** les coupleurs sont choisis parmi les métaphénylènediamines, les métaaminophénols, les métadiphénols et les coupleurs hétérocycliques.

19. Composition selon la revendication 18, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, 6-hydroxybenzomorpholine, la 2,6-dihydroxy 4-méthyl pyridine, le 1-H 3-méthyl pyrazole 5-one, le 1-phényl 3-méthyl pyrazole 5-one, et leurs sels d'addition avec un acide.

20. Composition selon l'une quelconque des revendications 17 à 19, **caractérisée par le fait que** le ou les coupleurs représentent de 0,0001 à 10 % en poids du poids total de la composition tinctoriale.

21. Composition selon la revendication 20, **caractérisée par le fait que** le ou les coupleurs représentent de 0,005 à 5 % en poids du poids total de la composition tinctoriale.

22. Composition selon l'une quelconque des revendications 7 à 21, **caractérisée par le fait que** les sels d'addition avec un acide sont choisis parmi les chlorhydrates, les bromhydrates, les sulfates, les citrates, les succinates, les tartrates, les lactates et les acétates.

23. Procédé de teinture d'oxydation des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 7 à 22, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée séparément, simultanément ou séquentiellement.

24. Procédé selon la revendication 23, **caractérisé par le fait que** l'agent oxydant est choisi parmi le peroxyde d'hydrogène, le peroxyde d'urée, les bromates de métaux alcalins, les persels, les peracides, et les enzymes.

25. Dispositif à plusieurs compartiments, ou "kit" de teinture à plusieurs compartiments, dont un premier compartiment renferme une composition tinctoriale telle que définie à l'une quelconque des revendications 7 à 22 et un second compartiment renferme une composition oxydante.

## Patentansprüche

1. Verbindungen der folgenden Formel (I) und deren Additionssalze mit einer Säure: 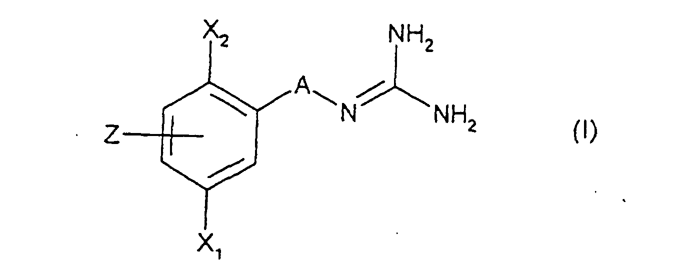 worin bedeuten
- X₁ und X₂ eine Hydroxygruppe, eine Gruppe -NHR₁ oder -NR₂R₂, mit der Maßgabe, dass X₁ und X₂ nicht gleichzeitig eine Hydroxygruppe bedeuten können;
- R₁ und R₂, die gleich oder verschieden sind, ein Wasserstoffatom, C₁₋₈-Alkyl, C₁₋₈-Monohydroxyalkyl, C₂₋₈-Polyhydroxyalkyl, C₂₋₈-Aminoalkyl, C₁₋₄-Monoalkyl-C₁₋₄-aminoalkyl, C₁₋₄-Dialkyl-C₁₋₄-aminoalkyl oder C₁₋₈-Halogenalkyl; wenn X₁ und/oder X₂ eine Gruppe -NR₁R₂ bedeutet, können R₁ und R₂ auch gemeinsam mit dem Stickstoffatom, an das sie gebunden sind, einen 4-, 5- oder 6-gliedrigen, aromatischen oder nichtaromatischen Ring bilden;
- R₁ und R₂ können auch unabhängig voneinander einen aromatischen oder nichtaromatischen, 4-, 5- oder 6-gliedrigen Ring bilden, wobei der Ring ein oder mehrere Heteroatome enthalten kann;
- A eine zweiwertige Gruppe, die unter -CH=N- und -CH₂-NH-ausgewählt ist;
- Z ein Wasserstoffatom, ein Halogenatom, einen 4-, 5- oder 6-gliedrigen, aromatischen oder nichtaromatischen Ring, C₁₋₈-Alkyl, C₁₋₈-Monohydroxyalkyl, C₂₋₈-Polyhydroxyalkyl, C₂₋₈-Aminoalkyl, C₁₋₄-Monoalkyl-C₁₋₄-aminoalkyl, C₁₋₄-Dialkyl-C₁₋₄-aminoalkyl, C₁₋₈-Halogenalkyl, Cyano oder eine Gruppe -BR₃, wobei B eine zweiwertige Gruppe ist, die unter den folgenden Gruppen ausgewählt ist;
―CH=N― ;
―CH₂-NH- ; 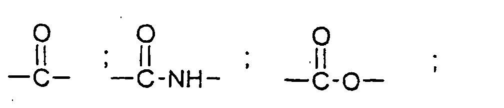 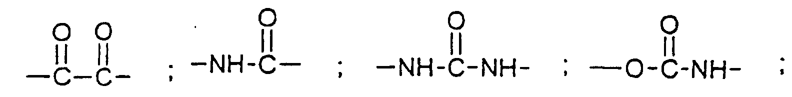
―S― ; 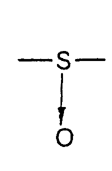
et 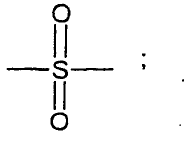
wobei R₃ C₁₋₈-Alkyl, C₁₋₈-Monohydroxyalkyl, C₂₋₈-Polyhydroxyalkyl, C₂₋₈-Aminoalkyl, C₁₋₄-Monoalkyl-C₁₋₄-aminoalkyl, C₁₋₄-Dialkyl-C₁₋₄-amionoalkyl oder C₁₋₈-Halogenalkyl bedeutet.

2. Verbindungen nach Anspruch 1, **dadurch gekennzeichnet, dass**, wenn R₁ und R₂ gemeinsam mit dem Stickstoffatom, an das sie gebunden sind, einen 4-, 5- oder 6-gliedrigen Ring bilden, oder R₁ und/oder R₂ einen 4-, 5- oder 6-gliedrigen Ring bilden oder Z einen aromatischen oder nichtaromatischen, 4-, 5- oder 6-gliedrigen Ring bedeutet, diese Ringe einen oder mehrere Substituenten tragen können, die unter den folgenden Gruppen ausgewählt sind: Hydroxy, Amido, Halogen, Cyano, D₁₋₈-Alkyl oder einen aromatischen oder nichtaromatischen C₄-, C₅- oder C₆-Ring.

3. Verbindungen nach Anspruch 1 oder 2, **dadurch gekennzeichnet, dass** sie ausgewählt sind unter:
- 5-Amino-2-hydroxy-benylidenamino-guanidin,
- 2-Amino-5-hydroxy-benylidenamino-guanidin,
- 2,5-Diamino-benzylidenamino-guanidin,
- 5-Dihydroxyethylamino-2-hydroxy-benylidenamino-guanidin,
- 5-Dimethylamino-2-hydroxy-benzylidenamino-guanidin,
- 2-Dihydroxyethylamino-5-hydroxy-benzylidenamino-guanidin,
- 2-Dimethylamino-5-hydroxy-benzylidenamino-guanidin,
- 2-Dihydroxyethylamino-5-amino-benzylidenamino-guanidin,
- 5-Dihydroxyethylamino-2-amino-benzylidenamino-guanidin,
- 5-Dimethylamino-2-amino-benzylidenamino-guanidin,
- 2-Dimethylamino-5-amino-benzylidenamino-guanidin,
- 2-N-Pyrrolidinyl-5-amino-benzylidenamino-guanidin,
- 5-N-Pyrrolidinyl-2-amino-benzylidenamino-guanidin,
und deren Additionssalzen mit einer Säure.

4. Verbindungen nach einem der vorhergehenden Ansprüche, **dadurch gekennzeichnet, dass** die Additionssalze der Verbindungen der Formel (I) mit einer Säure unter den Hydrochloriden, Hydrobomiden, Sulfaten, Citraten, Succinaten, Tartraten, Lactaten und Acetaten ausgewählt sind.

5. Verfahren zur Herstellung von Verbindungen der Formel (I) nach einem der Ansprüche 1 bis 4, das darin besteht, in einem organischen Lösungsmittel bei einer Temperatur im Bereich von 10 °C bis zur Rückflusstemperatur des Lösungsmittels das Aminoguanidin direkt an ein Benzaldehydderivat, dessen Substituenten der gewünschten Verbindung der Formel (I) entsprechen, zu kuppeln.

6. Verwendung der Verbindungen der Formel (I) nach einem der Ansprüche 1 bis 4 als Oxidationsbasen zum oxidativen Färben von Keratinfasern und insbesondere menschlichen Keratinfasern, wie dem Haar.

7. Zusammensetzung zum oxidativen Färben von Keratinfasern und insbesondere menschlichen Keratinfasern, wie dem Haar, **dadurch gekennzeichnet, dass** sie als Oxidationsbase in einem zum Färben geeigneten Medium mindestens eine Verbindung der Formel (I) nach einem der Ansprüche 1 bis 4 enthält.

8. Zusammensetzung nach Anspruch 7, **dadurch gekennzeichnet, dass** die Verbindung(en) der Formel (I) 0,0005 bis 12 Gew.-% des Gesamtgewichts der Farbmittelzusammensetzung ausmachen.

9. Zusammensetzung nach Anspruch 8, **dadurch gekennzeichnet, dass** die Verbindung(en) der Formel (I) 0,005 bis 6 Gew.-% des Gesamtgewichts der Farbmittelzusammensetzung ausmachen.

10. Zusammensetzung nach einem der Ansprüche 7 bis 9, **dadurch gekennzeichnet, dass** sie neben der Verbindung oder den Verbindungen der Formel (I) mindestens eine zusätzliche Additionsbase enthält, die unter den p-Phenylendiaminen, die von den Verbindungen der Formel (I) verschieden sind, den Bisphenylalkylendiaminen, p-Aminophenolen, die von den Verbindungen der Formel (I) verschieden sind, o-Aminophenolen und heterocyclischen Basen ausgewählt sind.

11. Zusammensetzung nach Anspruch 10, **dadurch gekennzeichnet, dass** die p-Phenylendiamine unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-pphenylendiamin, 2,5-Dimethyl-p-phenylendiamin, N,N-Dimethylp-phenylendiamin, N,N-Diethyl-p-phenylendiamin, N,N-Dipropylp-phenylendiamin, 4-Amino-N,N-diethyl-3-methyl-anilin, N,N-Bis(β-hydroxyethyl)-p-phenylendiamin, 4-N,N-Bis(β-hydroxyethyl)amino-2-methyl-anilin, 4-N,N-Bis(β-hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl-β-hydroxyethyl)-p-phenylendiamin, N-(β,γ-Dihydroxypropyl)-p-phenylendiamin, N-(4'-Aminophenyl)-p-phenylandiamin, N-Phenyl-p-phenylendiamin, 2-β-Hydroxyethyloxy-p-phenylendiamin, 2-β-Acetylaminoethyloxy-p-phenylendiamin, N-(β-Methoxyethyl)-p-phenylendiamin und deren Additionssalzen mit einer Säure ausgewählt sind.

12. Zusammensetzung nach Anspruch 10, **dadurch gekennzeichnet, dass** die Bisphenylalkylendiamine unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methyl-aminophenyl)-tetramethylendiamin, N,N'-Bis(ethyl)-N,N'bis(4'-amino-3'-methylphenyl)-ethylendiamin, 1,8-Bis(2,5-diaminophenoxy)-3,5-dioxaoctan und deren Additionssalzen mit einer Säure ausgewählt sind.

13. Zusammensetzung nach Anspruch 10, **dadurch gekennzeichnet, dass** die p-Aminophenole unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethylphenol, 4-Amino-2-fluor-phenol und deren Additionssalzen mit einer Säure ausgewählt sind.

14. Zusammensetzung nach Anspruch 10, **dadurch gekennzeichnet, dass** die heterocyclischen Basen unter den Pyridinderivaten, Pyrimidinderivaten und Pyrazolderivaten ausgewählt sind.

15. Zusammensetzung nach einem der Ansprüche 7 bis 14, **dadurch gekennzeichnet, dass** die zusätzliche(n) Oxidationsbase(n) 0,0005 bis 12 Gew.-% des Gesamtgewichts der Farbmittelzusammensetzung ausmachen.

16. Zusammensetzung nach Anspruch 15, **dadurch gekennzeichnet, dass** die zusätzliche(n) Oxidationsbase(n) 0,005 bis 6 Gew.-% des Gesamtgewichts der Farbmittelzusammensetzung ausmachen.

17. Zusammensetzung nach einem der Ansprüche 7 bis 16, **dadurch gekennzeichnet, dass** sie mindestens einen Kuppler und/oder mindestens einen Direktfarbstoff enthält.

18. Zusammensetzung nach Anspruch 17, **dadurch gekennzeichnet, dass** die Kuppler unter den m-Phenylendiaminen, m-Aminophenolen, m-Dihydroxybenzolen und heterocyclischen Kupplern ausgewählt sind.

19. Zusammensetzung nach Anspruch 18, **dadurch gekennzeichnet, dass** die Kuppler unter 2-Methyl-5-amino-phenol, 5-N-(β-Hydroxyethyl)amino-2-methyl-phenol, 3-Amino-phenol, 1,3-Dihydroxybenzol, 1,3-Dihydroxy-2-methyl-benzol, 4-Chlor-1,3-dihydroxybenzol, 2,4-Diamino-1-(β-hydroxyethyloxy)-benzol, 2-Amino-4-(β-hydroxyethylamino)-1-methoxy-benzol, 1,3-Diamino-benzol, 1,3-Bis(2,4-diaminophenoxy)propan, Sesamol, α-Naphthol, 2-Methyl-1-naphthol, 6-Hydroxy-indol, 4-Hydroxy-indol, 4-Hydroxy-N-methyl-indol, 6-Hydroxy-indolin, 6-Hydroxybenzomorpholin, 2,6-Dihydroxy-4-methyl-pyridin, 1-H-3-Methyl-pyrazol-5-on, 1-Phenyl-3-methyl-pyrazol-5-on und deren Additionssalzen mit einer Säure ausgewählt sind.

20. Zusammensetzung nach einem der Ansprüche 17 bis 19, **dadurch gekennzeichnet, dass** der oder die Kuppler 0,0001 bis 10 Gew.-% des Gesamtgewichts der Farbmittelzusammensetzung ausmachen.

21. Zusammensetzung nach Anspruch 20, **dadurch gekennzeichnet, dass** der oder die Kuppler 0,005 bis 5 Gew.-% des Gesamtgewichts der Farbmittelzusammensetzung ausmachen.

22. Zusammensetzung nach einem der Ansprüche 7 bis 21, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

23. Verfahren zum oxidativen Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 7 bis 22 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder vertrennt davon anschließend aufgetragen wird.

24. Verfahren nach Anspruch 23, **dadurch gekennzeichnet, dass** das Oxidationsmittel unter Wasserstoffperoxid, Harnstoffperoxid, Alkalimetallbromaten, Salzen von Persäuren, Persäuren und Enzymen ausgewählt ist.

25. Vorrichtung mit mehreren Abteilungen oder "Kit" zum Färben mit mehreren Abteilungen, wobei eine erste Abteilung eine Farbmittelzusammensetzung nach einem der Ansprüche 7 bis 22 enthält und eine zweite Abteilung eine oxidierende Zusammensetzung enthält.

## Claims

1. Compounds of formula (I) below, and the addition salts thereof with an acid: 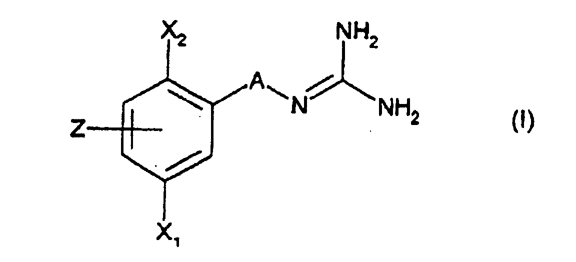 in which:
- X₁ and X₂ represent a hydroxyl radical, a radical -NHR₁ or -NR₁R₂, it being understood that X₁ and X₂ cannot simultaneously represent a hydroxyl radical;
- R₁ and R₂, which may be identical or different, represent a hydrogen atom, a C₁-C₈ alkyl radical, a C₁-C₈ monohydroxyalkyl radical, a C₂-C₈ polyhydroxyalkyl radical, a C₂-C₈ aminoalkyl radical, a mono (C₁-C₄)alkylamino(C₁-C₄)alkyl radical, a di(C₁-C₄)alkylamino(C₁-C₄)alkyl radical or a C₁-C₈ haloalkyl radical; when X₁ and/or X₂ represent(s) a radical -NR₁R₂, then R₁ and R₂ can also form together, in combination with the nitrogen atom to which they are attached, an aromatic or non-aromatic 4-, 5- or 6-membered ring;
- R₁ and R₂, independently of each other, may also represent an aromatic or non-aromatic 4-, 5- or 6-membered ring, the said ring possibly containing one or more heteroatoms;
- A represents a divalent radical chosen from -CH=N- and -CH₂-NH-;
- Z represents a hydrogen atom, a halogen atom, an aromatic or non-aromatic 4-, 5- or 6-membered ring, a C₁-C₈ alkyl radical, a C₁-C₈ monohydroxyalkyl radical, a C₂-C₈ polyhydroxyalkyl radical, a C₂-C₈ aminoalkyl radical, a mono (C₁-C₄)alkylamino(C₁-C₄)alkyl radical, a di (C₁-C₄) alkylamino (C₁-C₄) alkyl radical or a C₁-C₈ haloalkyl radical, a cyano radical or a group -BR₃ in which B represents a divalent radical chosen from the following radicals:
―CH=N― ;
―CH₂-NH- ; 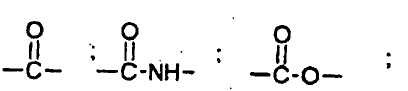 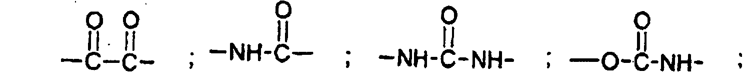
―S― ; 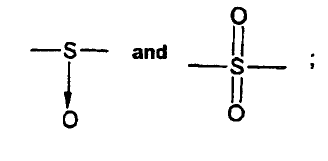
and in which R₃ represents a C₁-C₈ alkyl radical, a C₁-C₈ monohydroxyalkyl radical, a C₂-C₈ polyhydroxyalkyl radical, a C₂-C₈ aminoalkyl radical, a mono(C₁-C₄)alkylamino(C₁-C₄)alkyl radical, a di(C₁-C₄)alkylamino(C₁-C₄)alkyl radical or a C₁-C₈ haloalkyl radical.

2. Compounds according to Claim 1, **characterized in that** when R₁ and R₂ form, together with the nitrogen atom to which they are attached, a 4-, 5-or 6-membered ring, or when R₁ and/or R₂ represent a 4-, 5- or 6-membered ring, or when Z represents an aromatic or non-aromatic 4-, 5- or 6-membered ring, then the said rings may bear one or more substituents which may be chosen, for example, from the following groups: a hydroxyl radical, an amido radical, a halogen atom, a cyano radical, a C₁-C₈ alkyl radical, an aromatic or non-aromatic C₄, C₅ or C₆ ring.

3. Compounds according to Claim 1 or 2, **characterized in that** they are chosen from:
- 5-amino-2-hydroxy-benzylideneamino-guanidine,
- 2-amino-5-hydroxy-benzylideneamino-guanidine,
- 2,5-diamino-benzylideneamino-guanidine,
- 5-dihydroxyethylamino-2-hydroxy-benzylideneamino-guanidine,
- 5-dimethylamino-2-hydroxy-benzylideneaminoguanidine,
- 2-dihydroxyethylamino-5-hydroxy-benzylideneaminoguanidine,
- 2-dimethylamino-5-hydroxy-benzylideneaminoguanidine,
- 2-dihydroxyethylamino-5-amino-benzylideneaminoguanidine,
- 5-dihydroxyethylamino-2-amino-benzylideneaminoguanidine,
- 5-dimethylamino-2-amino-benzylideneamino-guanidine,
- 2-dimethylamino-5-amino-benzylideneamino-guanidine,
- 2-N-pyrrolidinyl-5-amino-benzylideneaminoguanidine,
- 5-N-pyrrolidinyl-2-amino-benzylideneaminoguanidine,
and the addition salts thereof with an acid.

4. Compounds according to any one of the preceding claims, **characterized in that** the addition salts with an acid of the compounds of formula (I) are chosen from the hydrochlorides, hydrobromides, sulphates, citrates, succinates, tartrates, lactates and acetates.

5. Process for preparing compounds of formula (I) as defined in any one of Claims 1 to 4, which consists in carrying out a direct coupling of the aminoguanidine with a benzaldehyde derivative whose substituents correspond to the compound of formula (I) which it is desired to obtain, in an organic solvent, at a temperature of between 10°C and the reflux temperature of the said solvent.

6. Use of the compounds of formula (I) as defined in any one of Claims 1 to 4, as oxidation bases for the oxidation dyeing of keratin fibres, and in particular of human keratin fibres such as the hair.

7. Composition for the oxidation dyeing of keratin fibres, and in particular of human keratin fibres such as the hair, **characterized in that** it comprises, as oxidation base, in a medium which is suitable for dyeing, at least one compound of formula (I) as defined in any one of Claims 1 to 4.

8. Composition according to Claim 7, **characterized in that** the compound(s) of formula (I) represent(s) from 0.0005% to 12% by weight relative to the total weight of the dye composition.

9. Composition according to Claim 8, **characterized in that** the compound(s) of formula (I) represent(s) from 0.005% to 6% by weight relative to the total weight of the dye composition.

10. Composition according to any one of Claims 7 to 9, **characterized in that**, in addition to the compound(s) of formula (I), it contains at least one additional oxidation base chosen from para-phenylenediamines other than the compounds of formula(I), bis(phenyl)alkylenediamines, para-aminophenols other than the compounds of formula (I), ortho-aminophenols and heterocyclic bases.

11. Composition according to Claim 10, **characterized in that** the para-phenylenediamines are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

12. Composition according to Claim 10, **characterized in that** the bis(phenyl)alkylenediamines are chosen from N,N'-bis(β-hydroxyethyl)-N,N'-bis(4'-aminophenyl)-1,3-diaminopropanol, N,N'-bis(β-hydroxy-ethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)-tetramethylenediamine, N,N'-bis(4-methylaminophenyl)tetramethylenediamine, N,N'-bis(ethyl)-N,N'-bis(4'-amino-3'-methylphenyl)ethylenediamine and 1,8-bis(2,5-diaminophenoxy)-3,5-dioxaoctane, and the addition salts thereof with an acid.

13. Composition according to Claim 10, **characterized in that** the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

14. Composition according to Claim 10, **characterized in that** the heterocyclic bases are chosen from pyridine derivatives, pyrimidine derivatives and pyrazole derivatives.

15. Composition according to any one of Claims 7 to 14, **characterized in that** the additional oxidation base(s) represent(s) from 0.0005% to 12% by weight relative to the total weight of the dye composition.

16. Composition according to Claim 15, **characterized in that** the additional oxidation base(s) represent(s) from 0.005% to 6% by weight relative to the total weight of the dye composition.

17. Composition according to any one of Claims 7 to 16, **characterized in that** it contains at least one coupler and/or at least one direct dye.

18. Composition according to Claim 17, **characterized in that** the couplers are chosen from meta-phenylenediamines, meta-aminophenols, meta-diphenols and heterocyclic couplers.

19. Composition according to Claim 18, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 2-methyl-1-naphthol, 6-hydroxyindole, 4-hydroxyindole, 4-hydroxy-N-methylindole, 6-hydroxyindoline, 6-hydroxybenzomorpholine, 2,6-dihydroxy-4-methylpyridine, 1H-3-methylpyrazol-5-one and 1-phenyl-3-methylpyrazol-5-one, and the addition salts thereof with an acid.

20. Composition according to any one of Claims 17 to 19, **characterized in that** the coupler(s) represent(s) from 0.0001% to 10% by weight relative to the total weight of the dye composition.

21. Composition according to Claim 20, **characterized in that** the coupler(s) represent(s) from 0.005% to 5% by weight relative to the total weight of the dye composition.

22. Composition according to any one of Claims 7 to 21, **characterized in that** the addition salts with an acid are chosen from the hydrochlorides, hydrobromides, sulphates, citrates, succinates, tartrates, lactates and acetates.

23. Process for the oxidation dyeing of keratin fibres, and in particular of human keratin fibres such as the hair, **characterized in that** at least one dye composition as defined in any one of Claims 7 to 22 is applied to the said fibres, and **in that** the colour is revealed at acidic, neutral or alkaline pH using an oxidizing agent which is added to the dye composition just at the time of use, or which is present in an oxidizing composition which is applied separately, simultaneously or sequentially.

24. Process according to Claim 23, **characterized in that** the oxidizing agent is chosen from hydrogen peroxide, urea peroxide, alkali metal bromates, persalts, peracids and enzymes.

25. Multi-compartment device or multicompartment dyeing "kit", a first compartment of which contains a dye composition as defined in any one of Claims 7 to 22, and a second compartment of which contains an oxidizing composition.
